(19) Europäisches Patentamt
European Patent Office
Office européen des brevets

(11) Veröffentlichungsnummer: **0 024 260**
**B1**

(12) # EUROPÄISCHE PATENTSCHRIFT

(45) Veröffentlichungstag der Patentschrift: **22.05.85**

(51) Int. Cl.⁴: **C 07 D 239/48,** A 01 N 43/54, C 07 D 239/38, C 07 D 239/56

(21) Anmeldenummer: **80810249.5**

(22) Anmeldetag: **11.08.80**

(54) **Neue Pyrimidin-Verbindungen, deren Herstellung, Zwischenprodukte dazu und herbizide Mittel, welche die Pyrimidinverbindungen als Wirkstoffe enthalten.**

(30) Priorität: **15.08.79 CH 7478/79**
**15.08.79 CH 7479/79**

(43) Veröffentlichungstag der Anmeldung:
**25.02.81 Patentblatt 81/8**

(45) Bekanntmachung des Hinweises auf die Patenterteilung:
**22.05.85 Patentblatt 85/21**

(84) Benannte Vertragsstaaten:
**BE CH DE FR GB IT LI NL**

(56) Entgegenhaltungen:
**EP - A - 0 000 681**

**CHEMICAL ABSTRACTS, Band 90, 1979, Seite 614, Nr. 54968y, Columbus, Ohio, US**
**CHEMICAL ABSTRACTS, Band 91, 1979, Seite 714, Nr. 57050z, Columbus, Ohio, US**
**Ariens, Drug Design, VI. S. 347 (1975)**

***Die Akte enthält technische Angaben, die nach dem*** **Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Hoegerle, Karl, Dr., St. Albananlage 19, CH-4052 Basel (CH)**
Erfinder: **Tobler, Hans, Dr., Baselmattweg 157, CH-4123 Allschwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Pyrimidin-Verbindungen, Verfahren zu ihrer Herstellung, herbizide und den Pflanzenwuchs regulierende Mittel, welche diese neuen Pyrimidin-Verbindungen als Wirkstoffe enthalten, sowie deren Verwendung zur selektiven Unkrautbekämpfung in Nutzpflanzenkulturen oder zur Hemmung des Pflanzenwachstums.

Die Erfindung betrifft ferner 5-Sulfinylpyrimidine und deren Herstellung, welche als Zwischenprodukte für die Herstellung der obigen Pyrimidinverbindungen oder auch von Farbstoffen verwendet werden.

Die Pyrimidin-Verbindungen entsprechen der Formel I

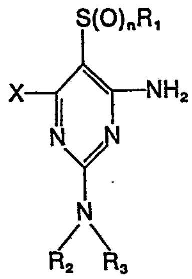

(I)

worin

n 1 oder 2, $R_1$ $C_1$–$C_4$ Alkyl, $R_2$ und $R_3$ Wasserstoff, $C_1$–$C_6$ Alkyl oder $C_3$–$C_6$ Cycloalkyl und X Chlor, Brom oder Fluor bedeuten.

Die Alkylgruppen umfassen alle darunterfallenden geradkettigen und verzweigten Gruppen.

Die Pyrimidin-Verbindungen der Formel I sind neue Verbindungen. Sie weisen ausgesprochen selektivherbizide Eigenschaften im allgemeinen auf und erweisen sich als besonders vorteilhaft zum Bekämpfen von Unkräutern in Kulturen von Nutzpflanzen, insbesondere in Kulturen von Mais aber auch von Soja, Baumwolle, Getreide wie Gerste, Hafer aber sowie auch von Zuckerrüben.

Bei genügend grosser Aufwandmenge ist jedoch auch totalherbizide Wirkung vorhanden. Die Anwendung kann sowohl im Vorauflauf- wie im Nachauflaufverfahren vorgenommen werden. Dabei können die Aufwandmengen in weiten Grenzen schwanken, z.B. zwischen 0,1 bis 10 kg Wirkstoff pro Hektare, vorzugsweise werden jedoch 0,5 bis 5 kg Wirkstoff pro Hektare eingesetzt.

Gute selektive Herbizidwirkung wurde teilweise aber schon bei Aufwandmengen von nur 0,25 kg pro Hektar beobachtet.

Die Verbindungen der Formel I zeigen auch den Pflanzenwuchs regulierende Wirkung, wie beispielsweise Wuchshemmung in Getreide. Diese Wuchshemmung betrifft nur das vegetative Wachstum. Man erhält so Getreide mit kürzeren dafür stärkeren Halmen, welche sich durch Wind und Unwetter nicht so leicht «legen» lassen. Das generative Wachstum bleibt erhalten; man kann durch die günstigeren Bedingungen sogar Ertragsteigerungen erzielen.

Die Pyrimidin-Verbindungen der Formel I sind neue Verbindungen. Pyrimidine mit herbizider oder sonst die Pflanzenphysiologie beeinflussender Wirkung sind in grosser Zahl bekannt geworden, als Beispiele seien genannt: J. prakt. Chemie 115, S. 292 (1927), DOS 2 006 145, DOS 2 356 644 oder neuerdings auch die Europäische Patentpublikation No. 681.

Die Herstellung der neuen Pyrimidin-Verbindungen geschieht in an sich bekannter Weise.

Eine erste Methode besteht darin, dass man ein 2,4,6-Trihalogen-4-alkylsulfinyl- oder alkylsulfonyl-pyrimidin der Formel II

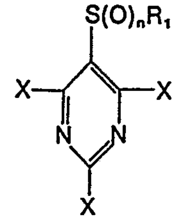

(II)

worin n, $R_1$ und X die unter Formel I gegebene Bedeutung haben, in einem den Reaktionsteilnehmern gegenüber inerten Lösungsmittel, in Gegenwart der säurebindenden Menge einer Base, mit der im Wesentlichen äquimolaren Menge des Amins der Formel III umsetzt,

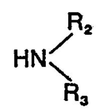

(III)

worin $R_2$ und $R_3$ die unter Formel I gegebene Bedeutung haben.

Man erhält so neben unerwünschten 4-Amino-2,6-dihalogen-5-alkylsulfinyl- oder -alkylsulfonyl-pyrimidin, das 2-Amino-4,6-dihalogen-5-alkylsulfinyl- oder alkylsulfonyl-pyrimidin der Formel IV

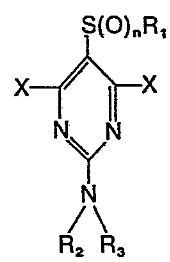

(IV)

worin n, $R_1$, $R_2$, $R_3$ und X die gegebene Bedeutung haben. Die Abtrennung erfolgt durch Säulechromatographie oder durch fraktionierte Kristallisation der beiden Isomeren. Die 2-Amino-Verbindungen sind leichter wasserlöslich als die 4-Amino-Isomeren. Die Abtrennung kann so gehandhabt werden, dass sie quantitativ erfolgt.

Das erste erfindungsgemässe Verfahren zur Herstellung der Pyrimidin-Verbindungen ist dadurch gekennzeichnet, dass man das so erhaltene 2-Amino-4,6-dihalogen-5-alkylsulfinyl oder -alkylsulfonyl-pyrimidin der obigen Formel IV, in einem inerten Lösungsmittel mit der doppeltmolaren Menge Ammoniak oder eines Amines umsetzt.

Diese Umsetzungen finden bei 0 °C bis ca. 150 °C statt, d. h. bis zum Siedepunkt des Lösungsmittels oder unter Druck in einem geschlossenen Gefäss.

Als Lösungsmittel kommen verschiedene in Frage, welche sich den Reaktionsteilnehmern gegenüber inert verhalten, wie beispielsweise Wasser, Methylenchlorid, Chloroform, Dioxan, Tetrahydrofuran, Dimethylformamid, Methanol, Äthanol, Isopropanol, Methyläthylketon, Diäthylketon, Toluol usw. Es kommen auch Gemische dieser Lösungsmittel untereinander oder mit Wasser in Frage.

Die 5-Alkylsulfonyl-2,4,6-trihalogen-pyrimidine der Formel II und ihre Herstellung sind beispielsweise in der DOS 2 819 837 oder dem französischen Patent No. 2 390 436 beschrieben. Dabei wird Barbitursäure in Gegenwart einer Base mit einem Alkylsulfonylhalogenid zur Alkylsulfonylbabitursäure umgesetzt, welche dann in einem Lösungsmittel mit N,N-Dimethylanilin und Phosphoroxychlorid behandelt wird.

Die 5-Alkylsulfinyl-2,4,6-trihalogen-pyrimidine der Formel II werden hergestellt, ausgehend von 2-Alkylthio-2,4,6-trihalogen-pyrimidinen, welche im J. of Medicinal Chemistry 18, S. 553 (1975) beschrieben sind, durch Oxydation mittels eines relativ milden Oxidationsmittels, wie beispielsweise Peressigsäure, 3-Chlorperbenzoesäure, Perbenzoesäure, Wasserstoffperoxid, Natriumperjodat, Perlaurinsäure, Jodbenzodichlorid, N-Chlorsuccinimid, N-Bromsuccinimid. Als Lösungsmittel kommen je nach dem angewendeten Oxidationsmittel, Methylchlorid, Chloroform, Essigsäure, Wasser etc. in Frage. Die Temperaturen dieser Oxidation liegen zwischen −50 °C und +50 °C. Ähnliche Oxidationsreaktionen sind beispielsweise in Tetrahedron Letters 1973, S. 2365 beschrieben, siehe dazu auch «Organic Compound of Bivalent Sulfur», Band 2, Seite 64 (Chemical Publishing Co. New York, 1960).

Gemäss einer weiteren Methode können die Pyrimidin-Verbindungen der Formel I hergestellt werden, indem man eine 2-Amino-4,6-dihydroxy-pyrimidin der Formel V

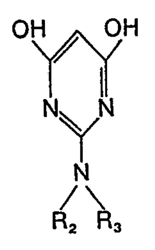

(V)

worin $R_2$ und $R_3$ die gegebene Bedeutung haben, indem man in alkalischem Milieu mit einem Alkylsulfonsäurehalogenid oder Alkylsulfinylsäurehalogenid der Formel VI umsetzt,

$$Hal{-}S(O)_nR_1 \qquad (VI)$$

worin Hal Chlor oder Brom bedeutet, während n und $R_1$ die gegebene Bedeutung haben. Das entstandene 2-Amino-4,6-dihydroxy-5-alkylsulfinyl- oder -alkylsulfonyl-pyrimidin wird durch Ansäuern des Reaktionsgemisches ausgefällt und isoliert und durch nachfolgendes Umsetzen mit einem starken Halogenierungsmittel wie Phosphoroxychlorid, Phosphoroxybromid, Sulfurylchlorid oder Sulfurylbromid gegebenenfalls in Gegenwart eines basischen Katalysators, vorzugsweise N,N-Dimethylanilin zum 2-Amino-4,6-dihalogen-5-alkylsulfinyl- oder -alkylsulfonyl-pyrimidin der obigen Formel IV chloriert. Es wird dann durch Umsetzen mit der doppelten molaren Menge Ammoniak oder einem Amin zu den erfindungsgemässen Pyrimidin-Verbindungen der Formel I umgesetzt.

Die Reaktionsbedingungen und Lösungsmittel für diese Methode sind im Wesentlichen dieselben, wie für die erste Herstellungsmethode. Die Ausgangsstoffe der Formel V sind bekannt und deren Herstellung beschrieben, siehe z. B. Journal of the Chemical Society, 1967, S. 2146 und Chemische Berichte 96, S. 2786 (1963).

Gemäss einer anderen Methode kann man schliesslich die Pyrimidin-Verbindungen der Formel I auch herstellen, indem man ein 2,4-Diamino-6-hydroxy-pyrimidin der Formel VII

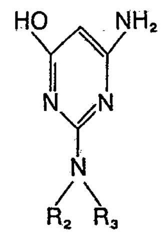

(VII)

worin $R_2$ und $R_3$ die gegebene Bedeutung haben, in alkalischem Milieu mit einem Alkylsulfinylhalogenid oder Alkylsulfonylhalogenid der obenstehenden Formel VI zum 2,4-Diamino-5-alkylsulfinyl- oder -alkylsulfonyl-6-hydroxy-pyrimidin der Formel VIII umsetzt,

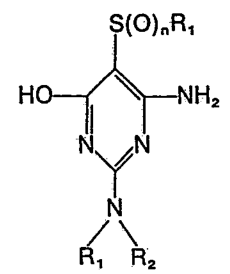

(VIII)

worin n, $R_1$, $R_2$ und $R_3$ die gegebene Bedeutung haben, und dieses Produkt durch Ansäuern des Reaktionsgemisches ausfällt und isoliert.

Die Herstellung der Pyrimidin-Verbindungen der Formel I ist dann dadurch gegeben, dass man das 2,4-Diamino-5-alkylsulfinyl- oder -alkylsulfonyl-6-hydroxy-pyrimidin der obenstehenden Formel VIII mit einem starken Halogenierungsmittel, wie Phosphoroxychlorid, Phosphoroxybromid, Sulfurylchlorid oder Sulfurylbromid umsetzt.

Diese Umsetzungen geschehen unter ähnlichen Bedingungen und in denselben Lösungsmitteln

wie diejenigen der ersten Herstellungsmethode. Die Ausgangsstoffe der Formel VII sind bekannt und beschrieben, siehe z.B. Journal of Organic Chemistry Bd. 28, S. 2672 (1963).

Die neuen Pyrimidin-Verbindungen der Formel I sind stabile Verbindungen, welche in üblichen organischen Lösungsmitteln, wie Alkoholen, Äthern, Ketonen, Dimethylformamid, Dimethylsulfoxyd etc. löslich sind. Sie sind relativ ungiftig und ihre Handhabung bedarf keiner besonderer Vorsichtsmassnahmen.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:

Stäubemittel, Streumittel, Granulate, Umhüllungsgranulate, Imprägniergranulate und Homogengranulate;

In Wasser dispergierbare Wirkstoffkonzentrate:

Spritzpulver (wettable powder), Pasten, Emulsionskonzentrate

Flüssige Aufarbeitungsformen:

Lösungen.

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen. So können die neuen Mittel ausser den genannten Verbindungen der allgemeinen Formel I z.B. Insektizide, Fungizide, Bakterizide, Fungistatika, Bakteriostatika, Nematozide oder weitere Herbizide zur Verbreiterung des Wirkungsspektrums enthalten.

In den folgenden Beispielen wird die Erfindung näher erläutert. Es wird die Herstellung der Pyrimidin-Verbindungen und der 5-Sulfinyl-pyrimidin Zwischenprodukte beschrieben, ferner diejenige gebrauchsfertiger Wirkstoffpräparate und -konzentrate und schliesslich auch die Versuchsanordnungen zur Feststellung der herbiziden und pflanzenwuchshemmenden Wirkung. Die Temperaturen sind in Grad Celsius gegeben, die Drücke in Millibar. Teile und Prozentangaben beziehen sich auf das Gewicht.

Beispiel 1

2,4-Diamino-5-methylsulfonyl-6-chlor-pyrimidin

In eine Lösung von 26,2 g (0,1 Mol) 5-Methylsulfonyl-2,4,6-trichlor-pyrimidin in 250 ml Methylenchlorid wird bei 5–10 °C trockener Ammoniak eingeleitet. Nach 2 Stunden bei 5–10 °C wird noch 16 Stunden bei Raumtemperatur ausgerührt, die Suspension filtriert und das Nutschengut mit Methylenchlorid und mit Wasser gewaschen. Die Kristalle werden getrocknet und aus 200 ml siedendem Monoglyme umkristallisiert. Man erhält 12 g 4,6-Diamino-5-methylsulfonyl-2-chlor-pyrimidin vom Smp. 280–281 °C. Das Monoglyme-Filtrat wird mit dem gleichen Volumen Äthanol verdünnt und die ausgefallenen Kristalle nach 2 Tagen abgesaugt. Man erhält so 2,5 g 2,4-Diamino-5-methylsulfonyl-6-chlorpyrimidin vom Smp. 256–258 °C.

Das als Ausgangsmaterial benötigte 5-Methylsulfonyl-2,4,6-trichlor-pyrimidin wurde wie folgt erhalten:

a) Zu einer Lösung von 11,9 g (0,09 Mol) Barbitursäure in 200 ml Wasser und 18 ml N-Natronlauge wird bei Raumtemperatur eine Lösung von 25 g (0,143 Mol) 96%igem Methansulfonsäureanhydrid in 25 ml Aceton zugetropft. Durch Zugabe von 30 ml 5 N-Natronlauge wird der pH bei 11,5–12 gehalten. Nach 4stündigem Rühren des Reaktionsgemisches bei Raumtemperatur ist die Reaktion beendet (pH konstant). Die erhaltene Suspension wird mit 20 ml konzentrierter Salzsäure versetzt (pH 1), und der entstandene Niederschlag wird abfiltriert. Man erhält 17,7 g des Natriumsalzes der 5-Methylsulfonyl-barbitursäure (86% d. Th.).

b) Zu 100 ml Phosphoroxychlorid werden bei Raumtemperatur langsam 12,5 ml (0,1 Mol) N,N-Dimethylanilin gegeben. Zu dieser Lösung werden bei Raumtemperatur 22,8 g (0,1 Mol) der oben erhaltenen Barbitursäure (Natriumsalz, oder eine entsprechende Menge der freien Säure) gegeben. Die Reaktionsmischung wird aufgeheizt, wobei bei etwa 55 °C langsam Chlorwasserstoffentwicklung beginnt. Die Suspension wird 15 bis 20 Stunden unter Rückfluss sieden gelassen. Anschliessend wird die trübe Lösung bei 25° bis 28 °C in Wasser gegossen und 20 Minuten gerührt. Der entstandene Niederschlag (17,6 g) wird lufttrocken in Methylenchlorid aufgenommen, die erhaltene Lösung wird mit Kohle und Natriumsulfat behandelt, dann wird klarfiltriert, und das Filtrat wird im Rotationsverdampfer bis zur Trockne eingeengt. Es werden 17,3 g fester Substanz erhalten. Diese werden in 100 ml Toluol gelöst und in der Lösung mit Aktivkohle behandelt, dann wird klarfiltriert, abgekühlt und auskristallisieren gelassen. Aus mehreren Kristallfraktionen erhält man schliesslich ca. 15,8 g (~ 60,4% der Theorie) 5-Methylsulfonyl-2,4,6-trichlor-pyrimidin, dessen Hauptfraktion einen Schmelzpunkt von 146/147 °C hat.

Beispiel 2

2,4-Diamino-5-methylsulfonyl-6-chlor-pyrimidin

Zur Suspension von 155,4 g (0,64 Mol) 2-Amino-4,6-dichlor-5-methylsulfonyl-pyrimidin in 750 ml Monoglyme werden innerhalb von 30 Minuten 130 ml 25%iger Ammoniak zugetropft. Die Suspension wird 5 Stunden auf 60° erwärmt und über Nach bei Raumtemperatur ausgerührt und filtriert. Man erhält 112,5 g der obigen Substanz vom Schmelzpunkt 250–252 °C. Durch Umkristallisieren aus Monoglyme lässt sich der Smp. auf 259–261 °C anheben.

Das Ausgangsmaterial wurde wie folgt erhalten:

a) 2-Amino-4,6-dihydroxy-5-methylsulfonyl-pyrimidin.

Zu einer Lösung von 218 g (1,71 Mol) 2-Amino-4,6-dihydroxypyrimidin in 2300 ml Wasser und 170 ml 30%iger Natronlauge werden innerhalb von 4 Stunden bei pH 11,5–12 und 5–10 °C (Eiskühlung), 296 g (2,58 Mol) 98%iges Methansulfochlorid zugetropft. Der pH-Wert von 11,5–12 wird durch gleichzeitiges Zutropfen von ca. 700 ml 5 N-Natronlauge gehalten. Das Reaktionsgemisch wird noch eine Stunde im Eisbad ausgerührt und filtriert. Man erhält 319,9 g (81,8% der Theorie) Natriumsalz der obigen Verbindung, woraus durch Umsatz mit Salzsäure die freie Säure isoliert werden kann.

b) 2-Amino-4,6-dichlor-5-methylsulfonyl-pyrimidin

In eine Lösung von 116 ml (0,91 Mol) Dimethylanilin in 1500 ml Phosphoroxychlorid werden 187 g (0,91 Mol) 2-Amino-4,6-dihydroxy-5-methylsulfonyl-pyrimidin zugestreut. Die Suspension wird langsam bis zum Rückfluss erwärmt und bei dieser Temperatur gehalten, bis die Salzsäureentwicklung beendet ist. Das Phosphoroxychlorid wird am Rotationsverdampfer abgezogen und der verbleibende honigartige Rückstand portionenweise auf 1000 ml Wasser gegossen. Durch Eiszugabe wird die Temperatur auf 20–25 °C gehalten. Die Lösung wird von braunem Schlamm geklärt und durch Zugabe von Natriumacetat der pH-Wert von ca. 0,5 auf 4,5 gepuffert. Das ausgeschiedene Dimethylanilin wird durch zweimaliges Ausschütteln mit Äther entfernt und die klare Wasserphase auf 40–45 °C erwärmt und 1 Stunde bei dieser Temperatur gehalten. Die Suspension wird über Nacht gerührt und bei 10 °C filtriert. Man erhält so 155,4 g des obigen Produktes, das bei 275° unter Zersetzung schmilzt. Beim längeren Stehen der Mutterlauge kristallisieren noch weitere 16,8 g des gleichen Materials.

Beispiel 3

2-Methylamino-4-amino-5-methylsulfonyl-6-chlor pyrimidin

Zu einer Lösung von 26,15 g (0,1 Mol) 5-Methylsulfonyl-2,4,6-trichlorpyrimidin in 300 ml Methylenchlorid werden unter Eiskühlung innerhalb ca. einer Stunde 18 ml (0,25 Mol) 25%iger Ammoniak-Lösung zugetropft. Die Suspension wird über Nacht bei 0–5 °C ausgerührt, dann mit gleichem Volumen Wasser versetzt, eine Stunde gut verrührt und über eine Sinternutsche filtriert. Das Nutschengut (18 g) wird in 80 ml Dimethylformamid heiss gelöst und mit 170 ml Methanol versetzt. Dabei kristallisieren 6,2 g Dünnschichtchromatographie-reines 4-Amino-5-methylsulfonyl-2,6-dichlorpyrimidin aus. Dieses wird in 150 ml Acetonitril gelöst und mit einer Lösung von 3,9 g 41%-igem wässrigem Methylamin in 50 ml Acetonitril versetzt. Die Suspension wird über Nacht bei 40° ausgerührt, dann im Rotationsverdampfer zur Trockene eingeengt und der Rückstand mit Wasser verrührt.

Aus dem wasserunlöslichen Anteil wird durch Kristallisation aus Essigsäure-Wasser (1 : 2) die Hauptmenge des unerwünschten 4,6-Isomeren entfernt. Die zur Trockne eingeengte Kristallisationsmutterlauge wird an 200 g Kieselgel mit dem Laufmittel Chloroform-Essigester (1 : 1) chromatographiert. Die zweite, langsamer wandernde Zone ist nach Infrarot und 13 C-NMR-Spektrum das gewünschte 2-Methylamino-4-amino-5-methylsulfonyl-6-chlor-pyrimidin vom Smp. 212–214 °C.

Beispiel 4

2,4-Diamino-5-methylsulfinyl-6-chlor-pyrimidin

20 g (0,08 Mol) 2,4,6-Trichlor-5-methylsulfinylpyrimidin, 12 g (0,7 Mol) gasförmiger Ammoniak und 100 ml Isopropanol werden im Autoklaven während 5 Stunden bei 100 °C behandelt. Das Reaktionsgut wird eingedampft und mit Wasser gewaschen. Der Schmelzpunkt des Rohproduktes ist 205–207 °C. Durch Umkristallisation aus Monoglyme erhält man 6,3 g Kristalle vom Smp. 221–223 °C sowie 4,3 g Kristalle vom Smp. 218–220 °C, insgesamt 10,6 g (64% Ausbeute). Gemäss 13 C-NMR-Spektrum handelt es sich um das gewünschte 2,4-Diamino-5-methylsulfinyl-6-chlor-pyrimidin.

Das als Ausgangsstoff benötigte 2,4,6-Trichlor-5-methylsulfinyl-pyrimidin wurde wie folgt erhalten:

a) Zu einer Lösung von 186,8 g (0,81 Mol) 2,4,6-Trichlor-5-methylthio-pyrimidin in 2 Liter Methylenchlorid werden bei −5 °C 156 g (90prozentig, 0,81 Mol) 3-Chlorperbenzoesäure in 1,5 Liter Methylenchlorid tropfenweise zugegeben. Man lässt bei Raumtemperatur während 15 Stunden rühren. Die Reaktionsmischung wird filtriert, 2mal mit 1N NaOH sowie 1mal mit $H_2O$ gewaschen, getrocknet (über $Na_2SO_4$), eingedampft und aus einem Essigester/Petroläthergemisch auskristallisiert. Die Ausbeute beträgt 147,3 g (74% der Theorie) Schmelzpunkt 126–128 °C.

In analoger Weise wurden folgende Pyrimidin-Verbindungen der Formel I hergestellt:

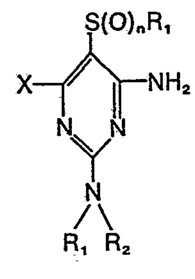

| Verb. No. | X | $S(O)_nR_1$ | $NR_1R_2$ | physikalische Daten |
|---|---|---|---|---|
| 1 | Cl | $SO_2CH_3$ | $NH_2$ | Smp. 256–8° |
| 2 | Cl | $SO_2CH_3$ | $NHCH_3$ | Smp. 212–4° |
| 3 | Cl | $SOCH_3$ | $NH_2$ | Smp. 221–3° |
| 4 | Cl | $SO_2CH_3$ | $N(CH_3)_2$ | Smp. 137–8° |
| 5 | Cl | $SO_2CH_3$ | $NHC_3H_7$iso | Smp. 122–3° |
| 6 | Cl | $SOCH_3$ | $NHCH_3$ | Smp. 215(Z)° |
| 8 | Cl | $SOCH_3$ | $N(CH_3)_2$ | Smp. 122–4° |
| 9 | Cl | $SOCH_3$ | $NHC_2H_5$ | Smp. 161–2° |
| 10 | Br | $SO_2C_2H_5$ | $NH_2$ | |
| 11 | Cl | $SO_2H_2H_5$ | $N(C_2H_5)_2$ | |
| 12 | Br | $SO_2CH_3$ | $N(CH_3)_2$ | |

| Verb. No. | X | $S(O)_nR_1$ | $NR_1R_2$ | physikalische Daten |
|---|---|---|---|---|
| 13 | F | $SO_2CH_3$ | $NH_2$ | Smp. 215–6° |
| 14 | Cl | $SO_2C_4H_9n$ | $NH_2$ | Smp. 220–2° |
| 15 | Cl | $SOCH_3$ | $NHC_3H_7iso$ | Smp. 133–5° |
| 17 | Cl | $SOCH_3$ | 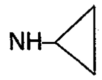 | Smp. 155–6° |
| 18 | Cl | $SOCH_3$ | $NHC_6H_{13}n$ | Smp. 100–02° |
| 19 | Cl | $SOCH_3$ | $NHC_4H_9t$ | Smp. 156–60° |
| 20 | F | $SOCH_3$ | $NH_2$ | Smp. 221–2° |

Ferner werden folgende Zwischenprodukte entsprechend den Formeln II, IV und VIII hergestellt:

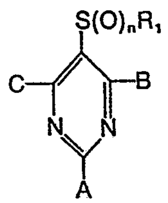

| $S(O)_nR_1$ | A | B | C | physikalische Konstante |
|---|---|---|---|---|
| $SOCH_3$ | Cl | Cl | Cl | Smp. 126–8° |
| $SO_2CH_3$ | $NH_2$ | OH | OH | Smp. >300° zers. |
| $SO_2CH_3$ | $NH_2$ | Cl | Cl | Smp. 275° zers. |
| $SO_2C_2H_5$ | Cl | Cl | Cl | Smp. 135–6 |
| $SO_2C_4H_9n$ | Cl | Cl | Cl | Smp. 71–2° |

Beispiel 5

Herstellung gebrauchsfertiger Wirkstoffpräparate und Wirkstoffkonzentrate

Spritzpulver

Zur Herstellung eines a) 70%igen und b) 10%igen Spritzpulvers werden folgende Bestandteile verwendet:

a) 70 Teile einer Pyrimidin-Verbindung der Formel I
5 Teile Natriumdibutylnaphthylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide;

b) 10 Teile Wirkstoff
3 Teile Gemisch der Natriumsalze von gesättigten Fettalkoholsulfaten,
5 Teile Naphthalinsulfonsäuren-Formaldehyd-Kondensat,
82 Teile Kaolin.

Die Pyrimidin-Verbindung wird auf die entsprechenden Trägerstoffe (Kaolin und Kreide) aufgezogen und anschliessend vermischt und vermahlen mit den übrigen Bestandteilen. Man erhält Spritzpulver von vorzüglicher Benetzbarkeit und Schwebefähigkeit. Aus solchen Spritzpulvern können durch Verdünnen mit Wasser Suspensionen von 0,1–8% Wirkstoff erhalten werden, die sich zur Unkrautbekämpfung in Pflanzenkulturen eignen.

Paste

Zur Herstellung einer 45%igen Paste werden folgende Stoffe verwendet:
45 Teile einer Pyrimidin-Verbindung der Formel I,
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylenglykol,
23 Teile Wasser.

Die Pyrimidin-Verbindung wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden
25 Teile einer Pyrimidin-Verbindung der Formel I,
10 Teile einer Mischung von Nonylphenolpolyoxyäthylen oder Calciumdidecylbenzolsulfonat,
10 Teile Cyclohexanon,
55 Teile Xylol

miteinander vermischt. Dieses Konzentrat kann mit Wasser zu Emulsionen auf geeignete Konzentrationen von z.B. 0,1% verdünnt werden. Solche Emulsionen eignen sich zur Bekämpfung von Unkräutern in Kulturpflanzungen.

Beispiel 6

Zum Nachweis der Wirkung als Herbizide (pre- und postemergent) dienen folgende Versuchsanordnungen:

Pre-emergente Herbizid-Wirkung (Keimhemmung)

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 Wirksubstanz pro Hektar. Die Saatschalen werden im Gewächshaus bei 22–25 °C und 50–70% rel. Luftfeuchtigkeit gehalten und der Versuch nach 3 Wochen ausgewertet.

Die Verbindungen 1, 2 und 3 zeigten in diesem Versuch ausgezeichnete Wirkung gegen die breitblättrigen und auch gegen die meisten grasartigen Unkräuter, während Kulturpflanzen wie Mais und zum Teil auch Weizen, Hirse, Reis, Soja und

Baumwolle nicht oder nur unbedeutend geschädigt wurden.

Post-emergente Herbizid-Wirkung (Kontaktherbizid)

Eine grössere Anzahl Unkräuter und Kulturpflanzen, sowohl monocotyle wie dicotyle, wurden nach dem Auflaufen (in 4- bis 6-Blattstadium) mit einer wässrigen Wirkstoffdispersion in Dosierungen von 0,5, 1, 2 und 4 kg Wirksubstanz pro Hektar auf die Pflanzen gespritzt und diese bei 24°–26 °C und 45–60% relativer Luftfeuchtigkeit gehalten. Mindestens 15 Tage nach der Behandlung wird der Versuch ausgewertet.

Auch in diesem Versuch zeigten die Verbindungen 1, 2 und 3 ausgezeichnete Wirkung gegen die breitblättrigen und die meisten der grasartigen Unkräuter, wobei die Kulturpflanze Mais, die Getreidearten Gerste, Hirse und Reis wie auch Baumwolle und Soja nicht oder erst mit höheren Aufwandmengen Wirkstoff geschädigt wurden.

Wuchshemmung bei Gräsern

In Kunststoffschalen mit Erde-Torf-Sand-Gemisch (6 : 3 : 1) wurden Samen der Gräser Lolium perenne, Poa pratensis, Festuca ovina und Cynodon dactylon ausgesät und nach Bedarf bewässert. Die aufgelaufenen Gräser wurden wöchentlich bis auf 4 cm Höhe zurückgeschnitten und 50 Tage nach der Aussaat und 1 Tag nach dem letzten Schnitt mit wässrigen Spritzbrühen eines Wirkstoffes der Formel I bespritzt. Die Wirkstoffmenge betrug umgerechnet 0,5 und 2,5 kg Aktivsubstanz pro Hektar. 21 Tage nach der Applikation wurde das Wachstum der Gräser beurteilt.

Wuchshemmung bei Getreide

In Kunststoffbechern mit sterilisierter Gartenerde wurden die Getreidearten Hordeum vulgare (Sommergerste) und Secale (Sommerroggen) im Gewächshaus angesät und nach Bedarf bewässert. Die Getreidesprösslinge werden 5 Tage nach Aussaat mit einer Spritzbrühe eines Wirkstoffes der Formel I besprüht. Die Wirkstoffmenge betrug umgerechnet 0,5 und 2,5 kg Aktivsubstanz pro Hektar. 10 und 21 Tage nach Applikation wurde das Wachstum des Getreides beurteilt.

Die erfindungsgemässen Wirkstoffe 1, 2 und 3 bewirken eine merkliche Wuchshemmung sowohl im Getreide wie auch bei Gräsern.

In einem weiteren Aspekt umfasst die Erfindung ebenfalls die Herstellung von 5-Sulfinyl-pyrimidinen, sowie diese Verbindungen als neue Substanzen. Sie dienen als Zwischenprodukte zur Herstellung der Pyrimidin-Verbindungen der Formel I oder auch von Farbstoffen.

Die 5-Sulfinyl-pyrimidine entsprechen der Formel IX

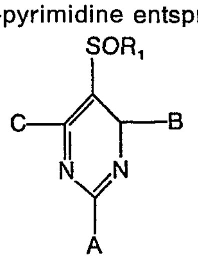

(IX)

worin A, B und C je ein Chlor, Brom oder Fluoratom oder eines davon auch eine Aminogruppe $-NR_2R_3$, $R_1$ $C_1$–$C_4$-Alkyl, $R_2$ und $R_3$ je Wasserstoff, $C_1$–$C_6$ Alkyl oder $C_3$–$C_6$ Cycloalkyl bedeuten.

Die Alkylgruppen umfassen alle geradkettigen und verzweigten Gruppen, welche die entsprechende Anzahl Kohlenstoffatome enthalten.

Die 5-Sulfinyl-pyrimidine sind neue Verbindungen. 5-Alkylthiopyrimidine ähnlicher Konstitution sind im J. of Medicinal Chemistry 18, S. 553 (1975) beschrieben worden, die Herstellung von 5-Alkylsulfonyl-trichlor-pyrimidin ist in der französischen Patentschrift No. 2 390 436 respektive der DOS 2 819 837 beschrieben worden.

Die 5-Alkylsulfinyl-2,4,6-trihalogen- oder 2,4,6-amino-dihalogen-pyrimidine der Formel I werden hergestellt, indem man ein 5-Alkylthio-2,4,6-trihalogenpyrimidin, respektive ein 5-Alkylthio-2,4,6-amino-dihalogen-pyrimidin der Formel X

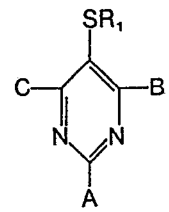

(X)

worin A, B, C und $R_1$ die gegebene Bedeutung haben, mit einem milden Oxidationsmittel oxydiert, und gegebenenfalls ein Halogenatom A, B oder C durch die Aminogruppe $HNR_1R_2$ ersetzt.

Die als Ausgangsmaterialien benötigten 2-Alkylthio-2,4,6-trihalogen-pyrimidine sind im J. of Medicinal Chemistry 18, S. 553 (1975) beschrieben. Beispiele milder Oxydationsmittel sind Peressigsäure, 3-Chlorperbenzoesäure, Perbenzoesäure, Wasserstoffperoxid, Natriumperjodat, Perlaurinsäure, Jodbenzodichlorid, N-Chlorsuccinimid, N-Bromsuccinimid. Als Lösungsmittel kommen je nach dem angewendeten Oxidationsmittel, Methylenchlorid, Chloroform, Essigsäure, Wasser etc. in Frage. Die Temperaturen dieser Oxidation liegen zwischen −50 °C und +50 °C. Ähnliche Oxidationsreaktionen sind beispielsweise in Tetrahedron Letters 1973, S. 2365 beschrieben, siehe dazu auch «Organic Compounds of Bilavent Sulfur», Band 2, Seite 64 (Chemical Publishing Co. New York, 1960).

Verbindungen der Formel I, worin A, B und C Brom- oder Fluoratome sind, lassen sich auch dadurch herstellen, dass man erfindungsgemäss erhaltene Verbindungen der Formel I, worin alle drei Chloratome sind, bis zum Ersatz von einem, zwei oder allen drei Chloratomen durch Brom- oder Fluoratome mit einem Bromierungs- oder Fluorierungsmittel, wie Phosphortribromid, wasserfreiem Fluorwasserstoff, Alkalimetallfluoriden oder Kaliumfluorosulfinat, umsetzt.

So können z.B. die Verbindungen der Formel I, worin A, B und C Chlor bedeuten und $SR_1$ die angegebene Bedeutung hat, in die Brom- oder Fluoranalogen übergeführt werden, indem man 5-Alkylsulfinyl-2,4,6-trichlorpyrimidin z.B. das 5-Methylsulfinyl-2,4,6-trichlorpyrimidin, durch Umsetzung mit Phosphortribromid, das auch als Lösungsmittel dienen kann, in das entsprechende 2,4,6-Tribrompyrimidin überführt, oder indem man die Chlor-Verbindungen durch Umsetzung mit wasserfreiem Fluorwasserstoff, mit Kaliumfluorosulfinat oder einem Alkalimetallfluorid unverdünnt oder in Gegenwart eines hochsiedenden aprotischen organischen Lösungsmittels in die entsprechende 2,4,6-Trifluorverbindung überführt. Geeignete Lösungsmittel für diese Umhalogenierung sind z.B. aromatische Kohlenwasserstoffe, wie Toluol, Benzol und Xylol; N,N-Dialkylamide von aliphatischen Monocarbonsäuren der vorerwähnten Art, wie N,N-Dimethylformamid und N,N-Dimethylacetamid; Dialkylsulfoxide, vor allem Dimethylsulfoxid; cyclische Äther und cyclische Amide, wie Tetrahydrofuran, Tetrahydropyran, N-Methyl-2-pyrrolidon und N-Acetyl-2-pyrrolidon; Hexamethylphosphorsäuretriamid (Hexametapol); N,N,N',N'-Tetramethylharnstoff, Tetrahydrothiophendioxid (Sulfolan).

Die Reaktionstemperaturen für die Umhalogenierung liegen zweckmässig zwischen 20 und 250 °C und bevorzugt zwischen 50 und 130 °C.

Die Herstellung der 5-Sulfinyl-pyrimidin-Verbindungen der Formel IX ist im Beispiel 4a veranschaulicht. Weitere in analoger Weise hergestellte 5-Sulfinyl-pyrimidin-Verbindungen sind in der folgenden Tabelle zusammengefasst.

| $SOR_1$ | A | B | C | physikalische Konstante |
|---|---|---|---|---|
| $SOCH_3$ | Cl | Cl | Cl | Smp. 126–8° |
| $SOCH_3$ | $NH_2$ | Br | Br | |
| $SOCH_3$ | $NH_2$ | Cl | Cl | Smp. 215–6° |
| $SOC_2H_5$ | Cl | Cl | Cl | |
| $SOC_4H_9$ | Cl | Cl | Cl | |
| $SOC_4H_7n$ | Cl | Cl | Cl | |
| $SOC_3H_7iso$ | Cl | Cl | Cl | |
| $SOC_4H_9tert.$ | Cl | Cl | Cl | |
| $SOCH_3$ | F | F | F | Smp. 46–48° |
| $SOCH_3$ | Br | Br | Br | |

**Patentansprüche**

1. Pyrimidin-Verbindungen der Formel I

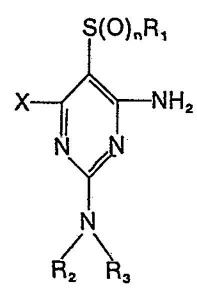

(I)

worin n 1 oder 2, $R_1$ $C_1$–$C_4$ Alkyl, $R_2$ und $R_3$ je Wasserstoff, $C_1$–$C_6$ Alkyl oder $C_3$–$C_6$ Cycloalkyl und X Chlor, Brom oder Fluor bedeuten.

2. Als Verbindung gemäss Anspruch 1: 4-Amino-2-methylamino-5-methylsulfonyl-6-chlor-pyrimidin.

3. Als Verbindung gemäss Anspruch 1: 2,4-Diamino-5-methylsulfonyl-6-chlorpyrimidin.

4. Als Verbindung gemäss Anspruch 1: 2-Methylamino-4-amino-5-methylsulfinyl-6-chlor-pyrimidin.

5. Verfahren zur Herstellung der Pyrimidin-Verbindungen der Formel I, Anspruch 1, dadurch gekennzeichnet, dass man ein 2-Amino-4,6-dihalogen-5-alkylsulfinyl- oder -alkylsulfonyl-pyrimidin der Formel IV

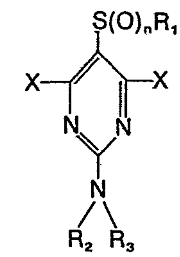

(IV)

worin n, $R_1$, $R_2$, $R_3$ und X die unter Formel I, Anspruch 1 gegebene Bedeutung haben, in einem inerten Lösungsmittel mit der doppeltmolaren Menge Ammoniak umsetzt.

6. Verfahren zur Herstellung der Pyrimidin-Verbindungen der Formel I, Anspruch 1, dadurch gekennzeichnet, dass man ein 2,4-Diamino-5-alkylsulfinyl- oder -alkylsulfonyl-6-hydroxy-pyrimidin der Formel VIII

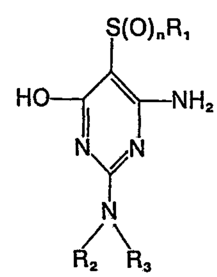

(VIII)

worin n, $R_1$, $R_2$ und $R_3$ die unter Formel I, Anspruch 1 gegebene Bedeutung haben, mit einem starken Halogenierungsmittel umsetzt.

7. Herbizide und den Pflanzenwuchs regulierende Mittel, dadurch gekennzeichnet, dass sie als Wirkstoff mindestens eine Pyrimidin-Verbindung der Formel I, Anspruch 1 enthalten.

8. Die Verwendung der Pyrimidin-Verbindungen der Formel I, Anspruch 1 oder sie enthaltender Mittel zur selektiven Bekämpfung von Unkräutern in Nutzpflanzenkulturen.

9. Die Verwendung der Pyrimidin-Verbindungen der Formel I, Anspruch 1 oder sie enthaltender Mittel zur selektiven Unkrautbekämpfung in Maiskulturen.

10. Die Verwendung der Pyrimidin-Verbindungen der Formel I, Anspruch 1 oder sie enthaltender Mittel zur Wuchshemmung in Getreidekulturen.

11. Verbindung der Formel IV

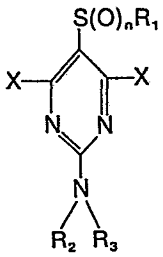

(IV)

worin
n 1 oder 2, $R_1$ $C_1$–$C_4$-Alkyl; $R_2$ und $R_3$ Wasserstoff, $C_1$–$C_6$-Alkyl oder $C_3$–$C_6$-Cycloalkyl; und X Chlor, Brom oder Fluor bedeuten.

12. Verbindung gemäss Anspruch 11, dadurch gekennzeichnet, dass n die Zahl 1 bedeutet.

13. Verfahren zur Herstellung einer Verbindung der Formel IV gemäss Anspruch 11, dadurch gekennzeichnet, dass man
a) eine Verbindung der Formel II

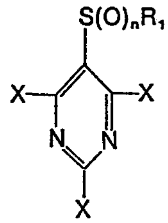

(II)

mit einem Amin der Formel III

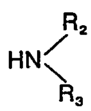

(III)

umsetzt
oder
b) eine Verbindung der Formel IIa

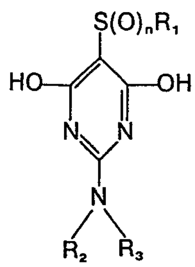

(IIa)

halogeniert, wobei in den Formeln II, IIa und III n, die Reste $R_1$–$R_3$ und X die unter Formel IV gemäss Anspruch 11 angegebenen Bedeutungen besitzen.

**Claims**

1. A pyrimidine of the formula I

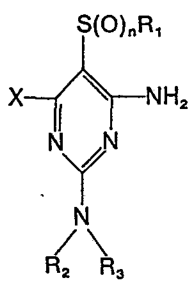

(I)

wherein n is 1 or 2, $R_1$ is $C_1$–$C_4$alkyl, each of $R_2$ and $R_3$ is hydrogen, $C_1$–$C_6$alkyl or $C_3$–$C_6$cycloalkyl, and X is chlorine, bromine or fluorine.

2. 4-Amino-2-methylamino-5-methylsulfonyl-6-chloropyrimidine according to claim 1.

3. 2,4-Diamino-5-methylsulfonyl-6-chloropyrimidine according to claim 1.

4. 2-Methylamino-4-amino-5-methylsulfinyl-6-chloropyrimidine according to claim 1.

5. A process for the production of a pyrimidine of the formula I according to claim 1, which process comprises reacting a 2-amino-4,6-dihalo-5-alkylsulfinylpyrimidine or -alkylsulfonylpyrimidine of the formula IV

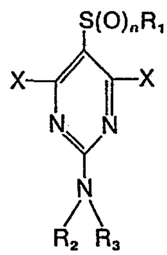

(IV)

wherein n, $R_1$, $R_2$, $R_3$ and X are as defined for formula I in claim 1, in an inert solvent, with twice the molar amount of ammonia.

6. A process for the production of a pyrimidine of the formula I according to claim 1, which process comprises reacting a 2,4-diamino-5-alkylsulfinyl- or -alkylsulfonyl-6-hydroxypyrimidine of the formula VIII

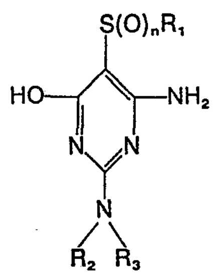

(VIII)

wherein n, $R_1$, $R_2$ and $R_3$ are as defined for formula I in claim 1, with a strong halogenating agent.

7. A herbicidal and plant growth-regulating composition which contains, as active ingredient, at least one pyrimidine of the formula I in claim 1.

8. A method of selectively controlling weeds in a crop of useful plants, which comprises applying to said crop a herbicidally effective amount of a pyrimidine of the formula I according to claim 1, or of a composition containing such a compound.

9. A method according to claim 8, wherein the crop is a maize crop.

10. A method of inhibiting growth in crops of cereals which comprises applying thereto an effective amount of a pyrimidine of the formula I according to claim 1, or of a composition containing such a compound.

11. A compound of formula IV

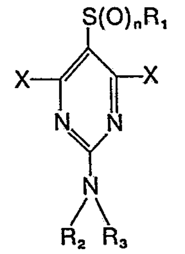

(IV)

wherein n is 1 or 2, $R_1$ is $C_1$–$C_4$alkyl, each of $R_2$ and $R_3$ is hydrogen, $C_1$–$C_6$alkyl or $C_1$–$C_6$cycloalkyl, and X is chlorine, bromine or fluorine.

12. A compound according to claim 11, wherein n is 1.

13. A process for the preparation of a compound of formula IV according to claim 11, which comprises

a) reacting a compound of formula II

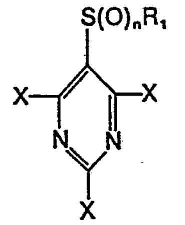

(II)

with an amine of formula III

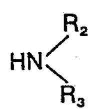

(III)

or

b) halogenating a compound of formula IIa

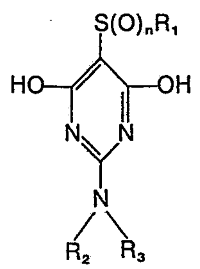

(IIa)

in which formulae II, IIa and III n, $R_1$ to $R_3$ and X are as defined for formula IV according to claim 11.

**Revendications**

1. Dérivés de la pyrimidine répondant à la formule I

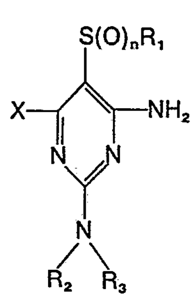

(I)

dans laquelle n est égal à 1 ou 2, $R_1$ représente un groupe alkyle en C1–C4, $R_2$ et $R_3$ représentent chacun un atome d'hydrogène, un groupe alkyle en C1–C6 ou cycloalkyle en C3–C6 et X représente le chlore, le brome ou le fluor.

2. En tant que composé selon la revendication 1: la 4-amino-2-méthylamino-5-méthylsulfonyl-6-chloropyrimidine.

3. En tant que composé selon la revendication 1: la 2,4-diamino-5-méthylsulfonyl-6-chloro-pyrimidine.

4. En tant que composé selon la revendication 1: la 2-méthylamino-4-amino-5-méthylsulfinyl-6-chloropyrimidine.

5. Procédé de préparation des dérivés de la pyrimidine répondant à la formule I de la revendication 1, caractérisé en ce que l'on fait réagir une 2-amino-4,6-dihalogéno-5-alkylsulfinyl- ou -alkylsulfonyl-pyrimidine de formule IV

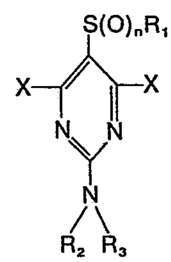

(IV)

dans laquelle n, $R_1$, $R_2$, $R_3$ et X ont les significations indiquées en référence à la formule I de la revendication 1, dans un solvant inerte avec la quantité molaire double d'ammoniac.

6. Procédé de préparation des dérivés de la pyrimidine répondant à la formule I de la revendication 1, caractérisé en ce que l'on fait réagir une 2,4-diamino-5-alkylsulfinyl- ou -alkylsulfonyl-6-hydroxy-pyrimidine de formule VIII

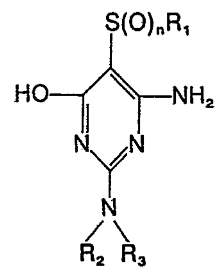

(VIII)

dans laquelle n, $R_1$, $R_2$ et $R_3$ ont les significations indiquées en référence à la formule I de la revendication 1, avec un agent halogénant puissant.

7. Produits herbicides et régulateurs de la croissance des végétaux, caractérisés en ce qu'ils contiennent en tant que substance active au moins un dérivé de la pyrimidine de formule I de la revendication 1.

8. L'utilisation des dérivés de la pyrimidine de formule I de la revendication 1, ou de produits en contenant pour la lutte sélective contre les mauvaises herbes dans les cultures de végétaux utiles.

9. L'utilisation des dérivés de la pyrimidine de formule I de la revendication 1 ou de produits en contenant pour la lutte sélective contre les mauvaises herbes dans les cultures de maïs.

10. L'utilisation des dérivés de la pyrimidine de formule I de la revendication 1 ou de produits en contenant pour l'inhibition de la croissance dans les cultures de céréales.

11. Composé de formule IV

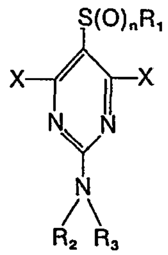

(IV)

dans laquelle
n est égal à 1 ou 2, $R_1$ représente un groupe alkyle en C1–C4; $R_2$ et $R_3$ représentent l'hydrogène, un groupe alkyle en C1–C6 ou cycloalkyle en C3–C6; et X représente le chlore, le brome ou le fluor.

12. Composé selon la revendication 11, caractérisé en ce que n est égal à 1.

13. Procédé de préparation d'un composé de formule IV selon la revendication 11, caractérisé en ce que
a) on fait réagir un composé de formule II

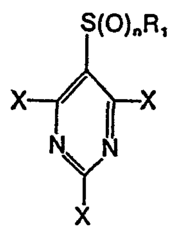

(II)

avec une amine de formule III

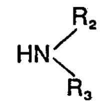

(III)

ou bien
b) on halogène un composé de formule IIa

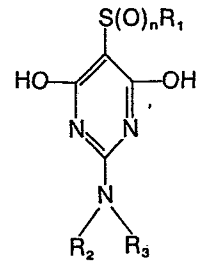

(IIa)

les symboles n, $R_1$ à $R_3$ et X ayant dans les formules II, IIa et III les significations indiquées en référence à la formule IV dans la revendication 11.